(19) Europäisches Patentamt
European Patent Office
Office européen des brevets

(11) Veröffentlichungsnummer: **0 217 735**
**A2**

(12) # EUROPÄISCHE PATENTANMELDUNG

(21) Anmeldenummer: 86730152.5

(22) Anmeldetag: 03.10.86

(51) Int. Cl.4: **C 07 D 457/12**
**A 61 K 31/48**

(30) Priorität: 04.10.85 DE 3535930

(43) Veröffentlichungstag der Anmeldung: 08.04.87 Patentblatt 87/15

(84) Benannte Vertragsstaaten: AT BE CH DE ES FR GB GR IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Huth, Andreas, Dr.
Kirchweg 55
D-1000 Berlin 38(DE)

(72) Erfinder: Sauer, Gerhard, Dr.
Königsbacher Zeile
D-1000 Berlin 28(DE)

(72) Erfinder: Wachtel, Helmut, Dr.
Asternplatz 2
D-1000 Berlin 45(DE)

(54) **Neue 2-substituierte Ergolinderivate.**

(57) Es werden neue 2-substituierte Ergolinyl-harnstoff- und -thioharnstoffderivate beschrieben sowie deren Verwendung als Pharmazeutika.

EP 0 217 735 A2

Croydon Printing Company Ltd

Die Erfindung betrifft neue 2-substituierte Ergolinderivate der allgemeinen Formel I, ihre Herstellung und ihre Verwendung als Arzneimittel.

Die erfindungsgemäßen Verbindungen haben die allgemeine Formel I

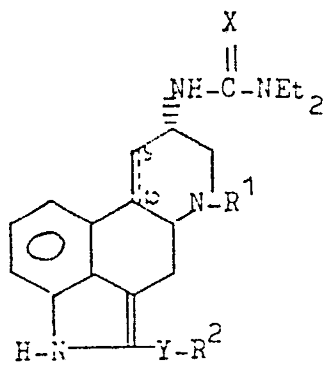

(I),

worin

X ein Sauerstoff- oder Schwefelatom bedeutet,

$R^1$ eine niedere Alkylgruppe ist,

Y ein Schwefelatom, eine $S-CH_2$-Gruppe, eine $C_2H_4$-, eine -CH=CH- oder eine -C≡C-Gruppe darstellt,

$R^2$ einen gegebenenfalls substituierten Aromaten bedeutet und

$C_9 \overset{---}{} C_{10}$ eine CC-Einfach- oder eine CC-Doppelbindung bedeutet und, wenn $C_9 \overset{---}{} C_{10}$ eine CC-Einfachbindung darstellt, das Wasserstoffatom in 10-Stellung $\alpha$-ständig ist sowie deren Säreadditionssalze, wobei, wenn X ein Sauerstoffatom, $R^1$ Methyl und $C_9 \overset{---}{} C_{10}$ eine Einfachbindung ist, $Y-R^2$ nicht $C_2H_4$-Phenyl oder C≡C-Phenyl bedeutet.

Unter den niederen Alkylresten versteht man solche mit bis zu 6 Kohlenstoffatomen, wobei $C_1$-$C_4$-Alkyle bevorzugt sind wie

BAD ORIGINAL

zum Beispiel Methyl, Ethyl, Isopropyl, n-Propyl, n-Butyl, Isobutyl und tert. Butyl.

Der Aromat $R^2$ enthält bis zu 6 Kohlenstoffatome, wobei ein oder mehrere Kohlenstoffatome durch Heteroatome wie Sauerstoff, Schwefel oder Stickstoff ersetzt sein können. Beispielsweise kommen als aromatische und heteroaromatische Reste in Frage; Phenyl, Pyridinyl, Thiophenyl, Furanyl, Pyrimidinyl, Imidazolyl, Pyrazolyl u.a.

Der Aromat kann in jeder beliebigen Position ein- oder mehrfach substituiert sein, beispielsweise mit niederem Alkyl, niederem Alkoxy, Nitril, Nitro, Amin, niederem Alkylamin, Carbonylamin, Halogen wie Fluor, Chlor, Brom oder Jod und anderen Substituenten.

Die niederen Alkylgruppen in $R^2$ haben insbesondere 1 - 2 Kohlenstoffatome.

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat,

BAD ORIGINAL

Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, ß-Hydroxybutyrat, Glycollat, Malat, Tartrat, Methansulfonat, Propansulfonat, Naphthalin-1-sulfonat oder Naphthalin-2-sulfonat.

Im Vergleich zu bekannten, in 2-Stellung unsubstituierten Ergolinen, wie zum Beispiel dem Lisurid oder dem Tergurid, sowie den aus der EP-A 160 842 bekannten 1,1-Diethyl-3-(2-phenyl-ethinyl-6-methyl-8α-ergolinyl)-harnstoff und dem analogen 2-Phenethyl-Derivat zeichnen sich die erfindungsgemäßen Verbindungen der Formel I durch eine zentral dopaminerge Wirksamkeit aus.

Die zentrale Dopaminrezeptorblockade von A wurde in einem Interaktionstest mit dem Dopaminrezeptoragonisten Apomorphin an Mäusen nach einmaliger i.p.-Vorbehandlung dargestellt (Parameter: Aufhebung der durch Apomorphin 5 mg/kg i.p. verursachten Hypothermie). Männliche NMRI-Mäuse wurden mit verschiedenen Dosen von A, die selbst nicht die Thermoregulation der Versuchstiere beeinflussen, bzw. mit Trägermedium vorbehandelt. 30 Minuten später erhielten alle Tiere Apomorphin 5 mg/kg i.p. 60 Minuten nach Gabe von A bzw. Trägermedium (= 30 Minuten nach Apomorphin) wurde die Rektaltemperatur mit Hilfe einer Thermosonde gemessen. Während die mit Trägermedium vorbehandelten Mäuse eine Hypothermie aufwiesen, war an mit A vorbehandelten Tieren der körpertemperatursenkende Effekt des Apomorphin dosisabhängig aufgehoben. (A = Substanz)

BAD ORIGINAL

Aufgrund dieser pharmakologischen Befunde können die erfindungsgemäßen Verbindungen als Neuroleptika zur Behandlung von Psychosen des schizophrenen Formenkreises verwendet werden.

Die Herstellung der Verbindungen der allgemeinen Formel I erfolgt nach an sich bekannten Methoden.

Beispielsweise kann man Verbindungen der allgemeinen Formel I mit Y in der Bedeutung eines Schwefelatoms oder einer $S-CH_2$-Gruppe darstellen, indem man in einem 2-Halogen-ergolinderivat in 1-Stellung eine Schutzgruppe einführt, anschliessend in 2-Stellung Halogen gegen Lithium austauscht und das so erhaltene 2-Lithiumergolinderivat der allgemeinen Formel II

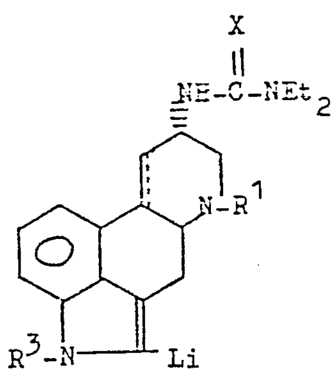

(II)

worin

X und $R^1$ die obige Bedeutung haben und $R^3$ eine übliche Schutzgruppe darstellt,

mit einem Schwefel-elektrophil umsetzt und die Schutzgruppe abspaltet und gegebenenfalls die Harnstoffe in die Thioharnstoffe überführt und/oder das physiologisch verträgliche Säureadditionssalz bildet.
Die Herstellung der Verbindungen der allgemeinen Formel II ist beispielsweise in der Europäischen Patentanmeldung Nr. 85104073.3 beschrieben.

BAD ORIGINAL

Als Schwefel-elektrophile seien bevorzugt organische Disulfide genannt wie beispielsweise gegebenenfalls ein- oder mehrfach substituierte aromatische Disulfide wie Diphenyldisulfide und Dibenzyldisulfide oder heteroaromatische Disulfide mit den oben angeführten heteroaromatischen Resten.

Die elektrophile Substitution wird bei tiefen Temperaturen, vorzugsweise zwischen - 110 °C bis - 50 °C in einem aprotischen Lösungsmittel vorgenommen.

In die Reaktion kann das Reaktionsgemisch der vorherigen Stufe ohne Aufarbeitung eingesetzt werden.

Als aprotische Lösungsmittel sind Ether oder Kohlenwasserstoffe geeignet wie beispielsweise Tetrahydrofuran, Dioxan, Diethylether, Toluol, Hexan u.a.

Als Schutzgruppe $R^3$ ist jede üblicherweise verwendete Schutzgruppe geeignet wie beispielsweise ein Acyl-, Arylsulfonyl- oder Silylrest, wobei der tri-Alkylsilylrest, - insbesondere die tert. Butyldimethylsilylgruppe, bevorzugt ist.

Die Abspaltung der Schutzgruppe erfolgt nach den üblichen Methoden durch Behandeln mit Säuren wie verdünnter Mineralsäure, Trifluoressigsäure oder anorganischen Basen wie KOH, NaOH oder Fluorid wie Tetrabutylammoniumfluorid in inerten Lösungsmitteln, zum Beispiel Wasser, Alkoholen, Kohlenwassersto fen u.a. bei Raumtemperatur.

Verbindungen der allgemeinen Formel I mit Y in der Bedeutung von $-C_2H_4-$, $-CH=CH-$ oder $-C\equiv C-$ werden dargestellt, indem man ein Ergolinderivat der allgemeinen Formel III

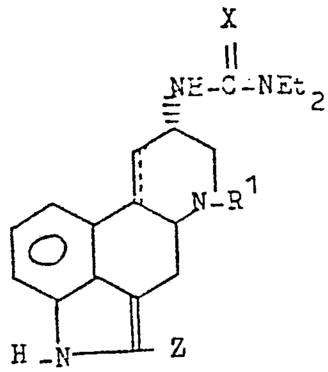

(III)

worin

$R^1$ und X die obige Bedeutung haben und Z ein Halogen oder -C≡CH bedeutet,

in Gegenwart eines Katalysators und einer Base mit einer Verbindung der Formel Halogen-$R^2$ bzw. H-C≡C-$R^2$ umsetzt, wobei jeweils ein Reaktionspartner eine halogenhaltige Verbindung darstellt und anschließend gegebenenfalls eine C≡C-Bindung partiell oder vollständig hydriert, die Harnstoffe in Thioharnstoffe überführt und/oder das Säureadditionssalz bildet. Die Herstellung der Verbindungen der allgemeinen Formel III ist beispielsweise in den EP-A 160 842, 56 358 und 141 387 beschrieben.

Die Umsetzung erfolgt bei Temperaturen von 0 °C bis 120 °C, bevorzugt 70 °C bis 100 °C, in einem aprotischen Lösungsmittel wie beispielsweise Dimethylformamid, N-Methylpyrrolidon, Tetrahydrofuran, Acetonitril oder Dioxan.

Als Katalysatoren werden bevorzugt Palladium-Verbindungen wie Palladiumsalze oder Palladium-Komplex-Verbindungen eingesetzt. Genannt seien zum Beispiel Palladium-II-acetat, trans-Dichlor-bis-(tri-o-tolyl-phosphin)-palladium(II) oder trans-Dichlor-bis-(triphenylphosphin)-palladium(II) und Palladium(O)-tetrakis-triphenylphosphin. Der Katalysator wird in einer Menge von 0,01 bis 0,1 Mol bezogen auf das eingesetzte 2-Halogen-ergolin angewendet.

BAD ORIGINAL

Ein Zusatz von Triarylphosphinen ist für die Reaktion förderlich. Für die Ethinylierung sind katalytische Mengen von Kupfer-I-jodid oder Kupfer-I-bromid angebracht.

Als Basen sind sekundäre und tertiäre Amine geeignet wie zum Beispiel Dimethylamin, Diethylamin. Piperidin, Triethylamin und Tri-n-butylamin.

Die halogenhaltige Verbindung enthält bevorzugt Jod oder Brom.
Die 2-Ethinyl-Verbindung kann durch katalytisch erregten Wasserstoff ganz oder partiell hydriert werden.

Die völlige Hydrierung wird in Gegenwart eines Katalysators wie beispielsweise Raney-Nickel oder Palladium auf verschiedenen Trägern wie Kohle bei Raumtemperatur gegebenenfalls unter erhöhtem Druck in einem inerten Lösungsmittel wie beispielsweise Alkoholen wie Methanol, Ethanol, Propanol, Ethern wie Dioxan, Diethylether, Säuren wie Eisessig u.a. durchgeführt.

Für die partielle Hydrierung zur Doppelbindung verwendet man als Katalysatoren beispielsweise mit Chinolin oder Pyridin, aber auch mit Blei vergiftete Palladiumsalze oder Palladium auf diversen Trägern (Lindlar Katalysatoren). Als Lösungsmittel dienen Alkohole oder Kohlenwasserstoffe.

Eine Umwandlung der Ethinylverbindung in Vinylverbindungen kann aber auch durch Anlagerung von metallorganischen Verbindungen wie zum Beispiel Diisobutylaluminiumhydrid und anschließende Hydrolyse durchgeführt werden. Als Lösungsmittel kommen Kohlenwasserstoffe oder Aether in Frage wie beispielsweise Hexan, Toluol, THF, Diethylether u.a.

Die Überführung der 8α-Harnstoffderivate in die entsprechenden Thione erfolgt durch Umsetzung mit Phosphoroxychlorid und nachfolgender Reaktion mit Kaliumxanthogenat. Die Reaktion wird bei tiefen Temperaturen mit zwischenzeitlicher Temperaturerhöhung in inerten Lösungsmitteln wie Ethern vorgenommen.

BAD ORIGINAL

Zur Bildung von Salzen werden die Verbindungen der Formel I in wenig Methanol oder Methylenchlorid gelöst und mit einer konzentrierten Lösung der gewünschten Säure in Methanol bei Raumtemperatur versetzt.

Alle Reaktionen werden im allgemeinen unter Schutzgasatmosphäre wie Argon oder Stickstoff teilweise unter erhöhtem Druck durchgeführt.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs, Stabilisierungs-, Netzmittel oder Emulgatoren, Salze zur Veränderung des osmotischen Drucks oder Puffer.

Die Herstellung der Ausgangsverbindungen ist bekannt oder erfolgt nach bekannten Methoden.

Die nachfolgenden Beispiele sollen das erfindungsgemäße Verfahren erläutern.

Mit Di-(4-methoxyphenyl)-disulfid erhält man 1,1-Diethyl-3-(2-(4-methoxyphenylthio)-6-methyl-8α-ergolinyl)-harnstoff.

Mit Di-(4-pyridyl)-disulfid erhält man 1,1-Diethyl-3-(6-methyl-2-(4-pyridylthio)-8α-ergolinyl)-harnstoff

Mit Di-(3-Fluorphenyl)-disulfid erhält man 1,1-Diethyl-3-(2-(3-fluorphenylthio)-6-methyl-8α-ergolinyl)-harnstoff

Aus 3-(2-Brom-1-(tert.-butyldimethylsilyl)-9,10-didehydro-6-methyl-8α-ergolinyl)-1,1-diethyl-harnstoff und Diphenyldisulfid wird der 3-(9,10-Didehydro-6-methyl-2-phenylthio-8α-ergolinyl)-1,1-diethyl-harnstoff in 43 % Ausbeute dargestellt.

Das gleiche Ausgangsmaterial wird mit folgenden Elektrophilen umgesetzt:

Dibenzyldisulfid gibt 3-(2-Benzylthio-9,10-didehydro-6-methyl-8α-ergolinyl)-1,1-diethyl-harnstoff in 52 % Ausbeute,

Di-(4-Chlorphenyl)-disulfid gibt 3-(2-(4-Chlorphenylthio)-9,10-didehydro-6-methyl-8α-ergolinyl)-1,1-diethyl-harnstoff,

Di-(3-methylphenyl)-disulfid gibt 3-(9,10-Didehydro-6-methyl-2-(methylphenylthio)-8α-ergolinyl)-1,1-diethyl-harnstoff,

Di-(4-Cyanophenyl)-disulfid gibt 3-(2-(4-Cyanophenylthio)-9,10-didehydro-6-methyl-8α-ergolinyl)-1,1-diethyl-harnstoff,

Di-(2-pyridyl)-disulfid gibt 3-(9,10-Didehydro-6-methyl-2-(2-pyrdridylthio)-8α-ergolinyl)-1,1-diethyl-harnstoff,

BAD ORIGINAL

Beispiel 1

1.1-Diethyl-3-(6-methyl-2-phenylthio-8α-ergolinyl)-harnstoff

Man stellt sich aus 4 ml wasserfreiem Toluol, 0,32 ml wasserfreiem, frisch destillierten Hexamethyldisilazan (1,5 mMol) und 0,85 ml n-Butyllithium in Hexan (1,4 mMol) bei 0 °C eine Lösung von Lithium-bis-(trimethylsilyl)-amid dar. Dazu gibt man die Lösung von 533 mg 3-(2-Brom-1-(tert.-butyldimethylsilyl)-6-methyl-8α-ergolinyl)-1,1-diethyl-harnstoff (1 mMol) in 50 ml wasserfreiem, frisch destillierten Toluol zu und rührt weitere 15 Minuten. Nach Zugabe von 0,8 ml destillierten Tetramethylethylendiamin wird auf - 90 °C abgekühlt, mit 4,5 ml tert.-Butyllithium (6,3 mMol) versetzt, zwei Minuten nachgerührt und die gebildete 2-Lithiumverbindung mit 2,2 g Diphenyldisulfid (10 mMol), gelöst in 10 ml wasserfreiem, frisch destillierten Toluol, bei - 70 °C zur Reaktion gebracht. Nach 30 Minuten Rühren bei - 70 °C wird Wasser zugegeben, die Toluolphase abgetrennt und die wässrige Phase mit Methylenchlorid ausgeschüttelt. Die organischen Phasen werden mit Natriumsulfat getrocknet und eingedampft, der Rückstand an Kieselgel chromatographiert. Die Ausbeute beträgt 523 mg 3-(1-(tert.-Butyldimehtylsilyl)-6-methyl-2-phenylthio-8α-ergolinyl)-1,1-diethyl-harnstoff ( 70 % d. Th.). Diese Substanz wird in 10 ml Methanol gelöst und mit 1 ml 14 n Kalilauge bei Raumtemperatur 20 Stunden gerührt. Man gibt Wasser zu dieser Mischung, rührt 30 Minuten im Eisbad und saugt den Niederschlag ab, Ausbeute 344 mg (82 % d. Th.). Durch Umkristallisation aus Methanol erhält man 229 mg des Endproduktes (Gesamtausbeute 51 %), $[\alpha]_D = + 20°$ (0,5 % in Chloroform).

In völlig analoger Weise wurde durch Verwendung von Dibenzyldisulfid der 3-(2-Benzylthio-6-methyl-8α-ergolinyl)-1,1-diethylharnstoff dargestellt, Ausbeute 73 %, $[\alpha]_D = - 55°$ (0,5 % in Chloroform).

Di-(3-nitrophenyl)-disulfid gibt 3-(9,10-Didehydro-6-methyl-2-(3-nitrophenylthio)-8α-ergolinyl)-1,1-diethyl-harnstoff,

Di-(4-Aminophenyl)-disulfid gibt 3-(2-(4-Aminophenylthio)-9,10-didehydro-6-methyl-8α-ergolinyl)-1,1-diethyl-harnstoff.

Aus 3-(2-Brom-1-(tert.-butyldimethylsilyl)-6-methyl-8α-ergolinyl)-1,1-diethyl-thioharnstoff und Diphenyldisulfid wird der 1,1-Diethyl-3-(6-methyl-2-phenylthio-8α-ergolinyl)-thioharnstoff dargestellt, Ausbeute 45 %, $[\alpha]_D = +60°$ (0,5 % in Chloroform).

Di-(4-Aminocarbonylphenyl)-disulfid gibt 3-(2-(4-Aminocarbonylphenylthio)-6-methyl-8α-ergolinyl)-1,1-diethyl-thioharnstoff

Di-(4-Fluorphenyl)-disulfid gibt 1,1-Diethyl-3-(2-(4-Fluorphenylthio)-6-methyl-8α-ergolinyl)-thioharnstoff.

Aus 3-(2-Brom-1-(tert.-butyldimethylsilyl)-6-n-propyl-8α-ergolinyl)-1,1-diethyl-harnstoff und Diphenyldisulfid wird der 1,1-Diethyl-3-(2-phenylthio-6-n-propyl-8α-ergolinyl)-harnstoff dargestellt.

Dibenzyldisulfid gibt 3-(2-(Benzylthio)-6-propyl-8α-ergolinyl)-1,1-diethyl-harnstoff.

Di-(4-pyridyl)-disulfid gibt 1,1-Diethyl-3-(6-n-propyl-2-(4-pyridylthio)-8α-ergolinyl)-harnstoff.

BAD ORIGINAL

Beispiel 2

3- [6-Methyl-2-(thien-2-ylethinyl)-8α-ergolinyl]-1.1-diethyl-harnstoff

72 mg 3-[6-Methyl-2-ethinyl-8α-ergolinyl]-1,1-diethylharnstoff werden in 2 ml Dimethylformamid mit 2 ml Triethylamin 1,5 mg Kupfer(I)jodid 0,069 ml 2-Jodthiophen und 4,8 mg Palladium(O)-tetraKistriphenylphosphin unter Argon 2 Stunden auf 80 °C erwärmt. Nach Eindampfen wird in Essigester aufgenommen und nacheinander mit verdünnter Ammoniaklösung und gesättigter Kochsalzlösung gewaschen. Nach Chromatographie über Kieselgel mit Essigester : Ethanol = 1 : 1 und Umkristallisation aus Ethanol/Hexan erhält man 26 mg 3-[6-Methyl-2-(thien-2-ylethinyl)-8α-ergolinyl]-1,1-diethylharnstoff vom Schmelzpunkt 252 - 256 °C.

In analoger Weise werden hergestellt:

3- [6-Methyl-2-(3-pyridinylethinyl)-8α-ergolinyl]-1,1-diethylharnstoff, Schmelzpunkt 226 - 229 °C, $[\alpha]_D$= + 124,6 ° (C = 0,215; Pyridin),

3-[2-(4-Methoxyphenylethinyl)-6-methyl-8α-ergolinyl]-1,1-diethylharnstoff, Schmelzpunkt 210 - 211 °C, $[\alpha]_D$= + 118 ° (C = 0,2; Pyridin).

Beispiel 3

3-[2-(4-Cyanophenylethinyl)-6-methyl-8α-ergolinyl]-1.1-diethylharnstoff

200 mg 3-[2-Jod-6-methyl-8α-ergolinyl]-1,1-diethylharnstoff werden in 4 ml DMF mit 4 ml Triethylamin, 4 mg Kupfer(I)jodid, 8 mg (Tetrakistriphenylphosphin)palladium(O) und 112 mg

BAD ORIGINAL

4-Cyanophenylacetylen unter Argon 2 Stunden auf 80 - 90 °C erwärmt. Nach Eindampfen wird in Essigester und gesättigter Natriumbicarbonatlösung verteilt und die organische Phase mit gesättigter Kochsalzlösung gewaschen. Nach Chromatographie über Kieselgel mit Methylenchlorid : Ethanol = 10 : 1 erhält man 87 mg 3-[2-(4-Cyanophenylethinyl)-6-methyl-8α-ergolinyl]-1,1-diethylharnstoff vom Schmelzpunkt 150 - 151 °C, $[\alpha]_D = + 139,5°$ (C = 0,2; Pyridin).

In analoger Weise werden dargestellt:

3-[2-(3-Methylphenylethinyl)-6-methyl-8α-ergolinyl]-1,1-diethylharnstoff,

3-[2-(2-Methylphenylethinyl)-6-methyl-8α-ergolinyl]-1,1-diethylharnstoff

3-[2-(4-Chlorphenylethinyl)-6-methyl-8α-ergolinyl]-1,1-diethylharnstoff

## Beispiel 4

**3-[2-(4-Methoxyphenethyl)-6-methyl-8α-ergolinyl]-1,1-diethylharnstoff**

50 mg 3-[2-(4-Methoxyphenethinyl)-6-methyl-8α-ergolinyl]-1,1-diethylharnstoff werden in 20 ml Ethanol mit einer Spatelspitze Raney-Nickel versetzt und bei Raumtemperatur und Wasserstoffnormaldruck 1 Stunde hydriert. Nach Abfiltrieren vom Katalysator wird aus Essigester/Hexan umkristallisiert. Man erhält 40 mg 3-[2-(4-Methoxyphenethyl)-6-methyl-8α-ergolinyl]-1,1-diethylharnstoff vom Schmelzpunkt 147 - 149 °C, $[\alpha]_D = + 4,2°$ (C = 0,2; Pyridin).

BAD ORIGINAL

Beispiel 5

3-(9.10-Didehydro-6-methyl-2-phenylethinyl-8α-ergolinyl)-1.1-diethylharnstoff

150 mg 3-(9,10-Didehydro-2-brom-6-methyl-8α-ergolinyl)-1,1-diethylharnstoff werden in 2 ml Dimethylformamid mit 5 mg Kupfer(I)jodid, 15 mg (Tetrakistriphenylphosphin)palladium(0), 4 ml Triäthylamin und 0,12 ml Phenylacetylen 6 Stunden auf 90 °C erwärmt; nach Filtration über Kieselgur wird nochmals mit 5 mg Kupfer(I)jodid, 15 mg Tetrakistriphenylphosphin-palladium(0) und 0,2 ml Phenylacetylen 3 Stunden auf 90 °C erwärmt, eingeengt, in Methylenchlorid sowie gesättigter Natriumbicarbonatlösung verteilt und die organische Phase mit gesättigter Kochsalzlösung gewaschen. Nach Chromatographie über Kieselgel mit Methylenchlorid : Ethanol = 10 : 1 und Umkristallisation aus Ethanol/Hexan erhält man 50 mg 3-(9,10-Didehydro-6-methyl-2-phenyl-ethinyl-8α-ergolinyl)-1,1-diethylharnstoff vom Schmelzpunkt 229 - 231 °C, $[\alpha]_D = + 195.8°$ (C = 0,23; Pyridin).

Beispiel 6

3-[6-Methyl-2-phenylethinyl-8α-ergolinyl]-1.1-diethylthioharnstoff

200 mg 3-[6-Methyl-2-phenylethinyl-8α-ergolinyl]-1,1-diethylharnstoff werden in 15 ml Methylenchlorid bei - 12 °C mit 0,13 ml Phosphoroxychlorid versetzt. In 1 Stunde läßt man den Ansatz auf Raumtemperatur erwärmen und rührt bei dieser Temperatur 16 Stunden. Nach Abziehen des Methylenchlorids wird unter Äther ausgerührt, abgesaugt und über KOH Plätzchen im Vakuum getrocknet. Es wird sodann mit einer Lösung von 245 mg

BAD ORIGINAL

Kaliumxanthogenat in 15 ml Acetonitril bei - 10 °C versetzt und 2 Stunden bei Raumtemperatur nachgerührt. Nach Eindampfen wird in Essigester/gesättigter Natriumhydrogenkarbonatlösung verteilt und die organische Phase mit gesättigter Kochsalzlösung gewaschen. Nach doppelter Chromatographie über Kieselgel zunächst mit dem Laufmittel Methylenchlorid/Ethanol wie 19 : 1 und beim zweiten Mal mit 10 : 1 und Umkristallisation aus Ethanol/Hexan/Essigester erhält man 60 mg 3-[6-Methyl-2-phenylethinyl-8α-ergolinyl]-1,1-diethylthioharnstoff vom Schmelzpunkt 219 - 220 °C.

$[\alpha]_D = + 195{,}8°$ (C = 0,25; Pyridin).

In analoger Weise wird hergestellt:

3-[6-Methyl-2-phenethyl-8α-ergolinyl]-1,1-diethylthioharnstoff.

Beispiel 7

3-(6-Methyl-2-phenylethenyl-8α-ergolinyl)-1,1-diethylharnstoff

200 mg 3-(6-Methyl-2-phenylethinyl-8α-ergolinyl)-1,1-diethylharnstoff werden zu einer bei Raumtemperatur und Normaldruck vorhydrierten Mischung von 150 mg Palladium/Bariumcarbonat (10 %ig) in 30 ml eines Lösungsmittelgemisches aus 10 Teilen Hexan, 2 Teilen Pyridin und 1 Teil Methanol gegeben und bis zum steilen Ansteigen der Kurve hydriert. Nach Abfiltrieren vom Katalysator wird eingeengt und über Kieselgel mit Essigester : Ethanol = 2 : 1 chromatographiert. Man erhält nach Umkristallisation 50 mg 3-(6-Methyl-2-phenethenyl-8α-ergolinyl)-1,1-diethylharnstoff als Gemisch der Z-und E-Verbindung.

BAD ORIGINAL

Beispiel 8

160 mg 3-(6-Methyl-2-phenethinyl-8α-ergolinyl)-1,1-diethylharnstoff werden unter Argon in 15 ml absolutem Toluol mit 3,5 ml einer circa 1,2 molaren DIBAH-Lösung in Toluol versetzt. Nach 10 Minuten Rühren bei Raumtemperatur wird 2 Stunden auf 80 °C erwärmt. Nach Abkühlen wird unter Schutzgas in eine 1 n wässrige Salzsäurelösung eingetragen, 10 Minuten gerührt, mit Ammoniaklösung alkalisch gestellt, über Kieselgur abgesaugt und mit Essigester gut nachgewaschen. Die organische Phase wird getrocknet, eingeengt und aus Essigester/Hexan umkristallisiert.
Man erhält 89 mg 3-(6-Methyl-2-E-phenethenyl-8α-ergolinyl)-1,1-diethylharnstoff vom $[\alpha]_D^{25} = + 123°$ (c = 0,2; Pyridin).

Patentansprüche

1.) 2-substituierte Ergolinderivate der allgemeinen Formel I

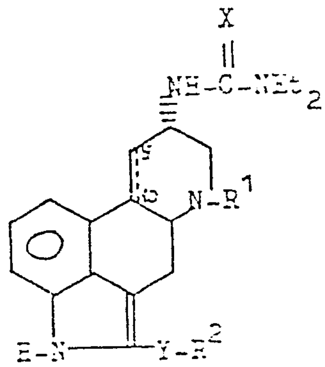

(I),

worin

X ein Sauerstoff- oder Schwefelatom bedeutet,

$R^1$ eine niedere Alkylgruppe ist,

Y ein Schwefelatom, eine $S-CH_2$-Gruppe, eine $C_2H_4$-, eine -CH=CH- oder eine -C≡C-Gruppe darstellt,

$R^2$ einen gegebenenfalls substituierten Aromaten bedeutet und

$C_9 \overset{...}{=} C_{10}$ eine CC-Einfach- oder eine CC-Doppelbindung bedeutet und, wenn $C_9 \overset{...}{=} C_{10}$ eine CC-Einfachbindung darstellt, das Wasserstoffatom in 10-Stellung α-ständig ist sowie deren Säureadditionssalze, wobei, wenn X ein Sauerstoffatom, $R^1$ Methyl und $C_9 \overset{...}{=} C_{10}$ eine Einfachbindung ist, Y-$R^2$ nicht -$C_2H_4$-Phenyl oder -C≡C-Phenyl bedeutet.

BAD ORIGINAL

2.) 1,1-Diethyl-3-(6-methyl-2-phenylthio-8α-ergolinyl)-harnstoff

3-(2-Benylthio-6-methyl-8α-ergolinyl)-1,1-diethyl-harnstoff

3-(9,10-Didehydro-6-methyl-2-phenylthio-8α-ergolinyl)-1,1-diethyl-harnstoff

3-(2-Benzylthio-9,10-didehydro-6-methyl-8α-ergolinyl)-1,1-diethyl-harnstoff

1,1-Diethyl-3-(6-methyl-2-phenylthio-8α-ergolinyl)-thioharnstoff

3-[6-Methyl-2-(thien-2-ylethinyl)-8α-ergolinyl]-1,1-diethyl-harnstoff

3-[6-Methyl-2-(3-pyridinylethinyl)-8α-ergolinyl]-1,1-diethyl-harnstoff

3-[2-(4-Methoxyphenylethinyl)-6-methyl-8α-ergolinyl]-1,1-diethyl-harnstoff

3-[2-(4-Cyanophenylethinyl)-6-methyl-8α-ergolinyl]-1,1-diethyl-harnstoff

3-[2-(4-Methoxyphenethyl)-6-methyl-8α-ergolinyl]-1,1-diethyl-harnstoff

3-(9,10-Didehydro-6-methyl-2-phenylethinyl-8α-ergolinyl)-1,1-diethyl-harnstoff

3-[6-Methyl-2-phenylethinyl-8α-ergolinyl]-1,1-diethylthioharnstoff

BAD ORIGINAL

0217735

3-(6-Methyl-2-phenylethenyl-8α-ergolinyl)-1,1-diethyl-harnstoff

.) Verfahren zur Herstellung der Verbindungen der allgemeinen Formel I, dadurch gekennzeichnet, daß man

a) 2-Lithiumergolinderivate der allgemeinen Formel II

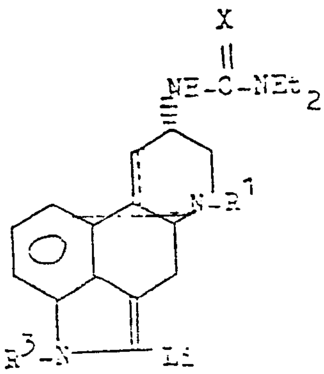

(II)

worin

X und $R^1$ die obige Bedeutung haben und $R^3$ eine übliche Schutzgruppe darstellt,

mit einem Schwefel-elektrophil umsetzt und die Schutzgruppe abspaltet und

b) Ergolinderivate der allgemeinen Formel III

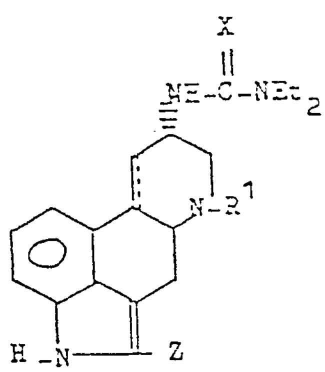

BAD ORIGINAL

worin

$R^1$ und X die obige Bedeutung haben und Z ein Halogen oder -C≡CH bedeutet,

in Gegenwart eines Katalysators und einer Base mit einer Verbindung der Formel Halogen-$R^2$ oder H-C≡C-$R^2$ umsetzt, wobei jeweils ein Reaktionspartner eine halogenhaltige Verbindung darstellt und gewünschtenfalls die C≡C-Bindung partiell oder vollständig hydriert und anschließend gegebenenfalls die Harnstoffe in die Thioharnstoffe überführt und/oder mit einer Säure das physiologisch verträgliche Säureadditionssalz bildet.

4.) Verwendung der Verbindungen gemäß Anspruch 1 und 2 als Arzneimittel.

BAD ORIGINAL